**Europäisches Patentamt**

**European Patent Office**

**Office européen des brevets**

(19)

(11) Veröffentlichungsnummer: **0 008 725**
**B1**

(12)

# EUROPÄISCHE PATENTSCHRIFT

(45) Veröffentlichungstag der Patentschrift:
26.08.81

(51) Int. Cl.³: **G 01 N 33/18**

(21) Anmeldenummer: **79103024.0**

(22) Anmeldetag: **18.08.79**

(54) **Verfahren und Messgerät zur Bestimmung des Sauerstoffeintrags in einen mit einem durchmischten fluiden Medium gefüllten Reaktor.**

(30) Priorität: **05.09.78 DE 2838621**

(43) Veröffentlichungstag der Anmeldung:
**19.03.80 Patentblatt 80/6**

(45) Bekanntmachung des Hinweises auf die Patenterteilung:
**26.08.81 Patentblatt 81/34**

(84) Benannte Vertragsstaaten:
**AT BE CH FR GB NL SE**

(56) Entgegenhaltungen:
**FR-A-2 196 294**
**FR-A-2 269 717**
**US-A-3 510 407**
**US-A-3 810 738**

(73) Patentinhaber: **Firma Edmund Bühler, Im Schelmen 11, D-7400 Tübingen 1-Weilheim (DE)**

(72) Erfinder: **Heinrich, Dietmar, Dipl.-Ing., Mozartweg 6, D-7054 Korb (DE)**
Erfinder: **Sekoulov, Ivan, Dr.-Ing., Köllestrasse 21, D-7000 Stuttgart 1 (DE)**

(74) Vertreter: **Möbus, Rudolf, Dipl.-Ing., Hindenburgstrasse 65, D-7410 Reutlingen (DE)**

**Verfahren und Messgerät zur Bestimmung des Sauerstoffeintrags in einen mit einem durchmischten fluiden Medium gefüllten Reaktor**

Die Erfindung betrifft ein Verfahren und ein Messgerät zur Bestimmung des Sauerstoffeintrags in einen mit einem durchmischten fluiden Medium gefüllten Reaktor. Ein solcher Reaktor ist beispielsweise das Belebungsbecken einer Kläranlage oder ein Bioreaktor.

Bei aeroben biologischen Vorgängen ist die ausreichende Sauerstoffversorgung der Belebungsbecken, Bioreaktoren und Fermenter, im folgenden kurz Reaktor genannt, für die Prozessführung von grosser Bedeutung.

Durch verschiedene technische Einrichtungen wird der von den Mikroorganismen veratmete Sauerstoff nachgeliefert. Die Geschwindigkeit des Sauerstoffeintrages in das fluide Medium ist von dem Belüftungssystem, der Reaktorgeometrie und dem Fluidum selbst abhängig. Demzufolge ist der Sauerstoffeintragswert bei veränderten einzelnen Parametern nicht direkt übertragbar und muss in situ bestimmt werden.

Die Sauerstoffeintragsmessungen in situ wurden bisher wie folgt durchgeführt:

a) unter Normbedingungen

Der Reaktor muss ausser Betrieb genommen und mit Reinwasser aufgefüllt werden. Durch Zugabe einer bekannten Menge von $Na_2SO_3$ wird der gelöste Sauerstoff (bei Anwesenheit von Katalysatoren z.B. Kobaltsalz) im Wasser chemisch gebunden. Erst nachdem die Sauerstoffkonzentration im Reaktor auf Null (mg $O_2$/l) gesunken ist, kann durch Einschalten des Belüftungsaggregates mit der Sauerstoffeintragsmessung begonnen werden. Während der Messung darf sich die Wassertemperatur, die Turbulenz im Reaktor und der Luftdruck nicht ändern. Die zeitliche Erhöhung der Sauerstoffkonzentration im Reaktor wird kontinuierlich gemessen bzw. registriert. Die Berechnung der Belüftungskonstanten erfolgt mit der Differentialgleichung (Gl. 1).

$$\frac{dc}{dt} = k_L a \, (c_s - c_t) \qquad\qquad \text{(Gl. 1)}$$

$c_s$   (mg $O_2$/l)   Sauerstoffsättigungswert des Reinwassers

$c_t$   (mg $O_2$/l)   Sauerstoffgehalt des Wassers zur Zeit t

$\frac{dc}{dt}$   (mg $O_2$/l)   Änderung des Sauerstoffgehaltes

$k_L a$   (h$^{-1}$)    Belüftungskonstante

Der Sauerstoffeintragswert OC lässt sich mit Hilfe von (Gl. 2) ermitteln.

$$OC = k_L a \cdot c_s \qquad \text{(Gl. 2)}$$

$k_L a$   (h$^{-1}$)    Belüftungskonstante

$c_s$   (mg $O_2$/l)   Sauerstoffsättigungswert des Reinwassers

b) unter Betriebsbedingungen

(Veröffentlichung des Instituts für Stadtbauwesen der TH Braunschweig Heft 1 Jahrgang 1967 Seite 23 ff; 39 ff; 42 ff und 74 ff; Titel «Ermittlung der Sauerstoffzufuhr von Abwasserbelüftern unter Betriebsbedingungen». Verfasser: Kayser, R.)

b1) Standversuch

Der Reaktor muss ausser Betrieb genommen werden, d.h. die Abwasserzufuhr und die Schlammrückführung werden unterbrochen. Der Reaktor wird jedoch nicht wie im Fall a (Normbedingungen) mit Reinwasser gefüllt, sondern bleibt mit dem vorhandenen Abwasserbelebtschlammgemisch gefüllt. Um eine geringere Atmungsaktivität der Mikroorganismen zu haben, bleibt die Belüftung für weitere 3–5 Stunden eingeschaltet. Nach Ausschalten des Belüftungsaggregates wird der im fluiden Medium gelöste Sauerstoff von den Mikroorganismen bis auf 0 mg/l verzehrt. Mit Einschalten des Belüftungsaggregates fängt die Sauerstoffeintragsmessung an. Die Erhöhung der Sauerstoffkonzentration im fluiden Medium wird gemessen und registriert (wie im Fall a). Dadurch, dass die Mikroorganismen im Reaktor ständig einen Teil des Sauerstoffes gleich veratmen, stellt sich ein Sauerstoffsättigungswert c* (hier virtuelle Sättigung genannt) kleiner als der tatsächliche Sättigungswert $c'_s$ ein. Daraus ist ersichtlich, dass für die Lösung der Aufgabe neben den gemessenen virtuellen Sauerstoffsättigungswerten noch die Atmungsaktivitäten der Mikroorganismen (Belebtschlammes) ($A_o$ (mg $O_2$/l·h) bestimmt werden müssen.

Die Atmungsaktivitätsmessung wurde bislang ausserhalb des Reaktors durchgeführt.

Für die Berechnung der Belüftungskonstanten wird die erweiterte (Gl. 3) mit dem Atmungsaktivitäts-Term benützt.

$$\frac{dc}{dt} = k_L a \, (c_s^* - c_t') - A_o \qquad\qquad \text{(Gl. 3)}$$

$A_o$   (mg $O_2$/l·h) Sauerstoffzehrung

$k_L a$   (h$^{-1}$)    Belüftungskonstante für Belebtschlamm

$c_s^*$   (mg $O_2$/l)   virtueller Sauerstoffsättigungswert, der sich bei konstanter Atmung und konstanter Sauerstoffzufuhr im Becken einstellt.

$c_t'$   (mg $O_2$/l)   Sauerstoffgehalt im Belebungsbecken zur Zeit t.

Zwischen dem Sauerstoffsättigungswert $c_s^*$ besteht folgender Zusammenhang:

$$c_s' = c_s^* + \frac{A_o}{k_L a} \qquad\qquad \text{(Gl. 4)}$$

Der Sauerstoffeintragswert OC′ lässt sich mit Hilfe von (Gl. 5) ermitteln.

$$OC' = k_L a \cdot c_s'$$
$$\text{(Gl. 5)}$$

OC′   (mg $O_2$/l·h) Sauerstoffzufuhr im Abwasserbelebtschlammgemisch

$c'_s$   (mg $O_2$/l)   Sauerstoffsättigungswert für Belebtschlamm

b2) Durchlaufversuch

Hier werden Abwasser- und Rücklaufschlammzugabe nicht unterbrochen. Der Sauerstoffgehalt im Belebungsbecken wird nach jeder Belastungsänderung gemessen. Daneben wird der Sauerstoffgehalt des Abwassers und Rücklaufschlammes bestimmt. Die Auswertung erfolgt mit Hilfe der vom Schreiber über die Zeit aufgezeichneten Sauerstoffgehaltswerte. Die Atmungsaktivitätsvermessungen werden weiterhin ausserhalb des Reaktors durchgeführt. Dazu ist es notwendig, im Labormessstab (Reaktionsgefäss) dasselbe Mischungsverhältnis zwischen Belebtschlamm und Abwasser (Belastung) wie im Reaktor zu erreichen. Diese komplizierte Messmethode ist ständig mit Fehlern behaftet.

Für die Bestimmung der Belüftungskonstanten wird folgende erweiterte (Gl. 6) verwendet:

$$k'_L a (c'_s - c^*_s) = A_o + q_A (c^*_s - c_A) + q_{RS} (c^*_s - C_{RS})$$
$$(Gl. 6)$$

$c_A$   (mg $O_2$/l)   Sauerstoffgehalt im Zulauf
$c_{RS}$   (mg $O_2$/l)   Sauerstoffgehalt im Rücklaufschlamm
$q_A$   ($m^3/m^3 \cdot h$)   Abwasserraumbeschickung
$q_{RS}$   ($m^3/m^3 \cdot h$)   Rücklaufschlammraumbeschickung

Reinwasserversuche sind sehr zeitaufwendig und teuer. Sie können wegen der Aufsalzung des Wassers mit $Na_2SO_4$ nicht beliebig oft ohne Messignalbeeinflussung wiederholt werden. Die Belebungsanlage muss auch für mindestens einen Tag aus dem Betrieb herausgenommen werden. Temperatur und Luftdruckveränderungen während des Versuches sind oft unvermeidbar. Bei Sauerstoffeintragsmessungen unter Betriebsbedingungen werden die Atmungsaktivitäten des Belebtschlammes ($A_o$ (mg $O_2$/l$\cdot$h) ausserhalb des Reaktors gemessen. Dadurch, dass die Atmung der Mikroorganismen je nach Belastung schon nach 20 min. nur noch 50% des Ausgangswertes betragen kann, sind diese Messungen mit Fehlern behaftet. Zudem bleiben die Temperaturunterschiede zwischen Reaktor und Messraum mit ihrem Einfluss oft unberücksichtigt.

Im Durchlaufversuch (b2) ist die Erfassung der Atmung des Belebtschlammes durch die Simulierung der Raumbelastung im Reaktionsgefäss (Labormessstab) ungenau. Es ist notwendig, dass die Versuche bis zum 99% Wert der Sauerstoffsättigung (Versuchsendwert) durchgeführt werden. Eine genaue Versuchsauswertung wird sonst unmöglich bzw. das Versuchsergebnis mit unzulässig hohen Fehlern behaftet.

Unabhängig davon wird bei Sauerstoffgehaltsmessungen in Reinwaser wie auch in Abwasser immer wieder festgestellt, dass aufsteigende Luftblasen an der Sauerstoffelektrode hängenbleiben. Ausserdem wird die Elektrode oft infolge zu geringer Turbulenzen im Reaktor nicht mit der nötigen Geschwindigkeit angeströmt. Beides führt zu Messfehlern. Wie daraus zu entnehmen ist, müssen die Sauerstoffeintrags- und Atmungsaktivitätsmessmethoden als noch nicht optimal angesehen werden.

Der Erfindung liegt die Aufgabe zugrunde, die bei der Sauerstoffeintragsbestimmung bislang bestehenden Schwierigkeiten hinsichtlich der mikrobiellen Respirationsmessung, der Versuchsdauer, des Messaufwandes, der Messgenauigkeit und der Betriebsbeeinträchtigung zu vermindern.

Die gestellte Aufgabe wird erfindungsgemäss dadurch gelöst, dass im Betrieb des Reaktors abwechselnd der Sauerstoffgehalt des im Reaktor enthaltenen fluiden Mediums und die Sauerstoffgehaltsänderung eines umlaufenden Teiles des fluiden Mediums gemessen werden. Das Verfahren hat den grossen Vorteil, dass der Reaktor zur Bestimmung des Sauerstoffeintrags nicht ausser Betrieb genommen werden muss und bei einer Kläranlage somit der Abwasserzulauf sowie der Schlammrücklauf nicht unterbrochen werden müssen. Dabei können vorteilhafterweise beide Messungen bei konstanter und über einem Minimalwert liegender Anströmung einer Sauerstoffelektrode durchgeführt werden, wodurch die Genauigkeit der Sauerstoffkonzentrationsmessungen und die Vergleichbarkeit der Messergebnisse begünstigt werden. Im umlaufenden Teil des fluiden Mediums wird durch Mikroben eine Abnahme der Sauerstoffkonzentration bewirkt, die es zu ermitteln gilt. Wenn die Sauerstoffkonzentration beim Abschliessen einer Bestimmung unter einem bestimmten Wert, z.B. 4 mg $O_2$/l liegt, kann eine genau definierte Menge an Reinsauerstoff oder $H_2O_2$ zugegeben werden, so dass die Konzentration des gelösten Sauerstoffes des umlaufenden Teiles des fluiden Mediums nicht über einen Wert von beispielsweise 10 mg $O_2$/l hinausgeht.

Durch die abwechselnde Messung des Sauerstoffgehaltes des im Reaktor enthaltenen fluiden Mediums und der Sauerstoffgehaltsänderung eines Teiles des im Reaktor enthaltenen fluiden Mediums lässt sich auf noch darzulegende Weise die erforderliche Sauerstoffzufuhr in den Reaktor zur Aufrechterhaltung eines optimalen Umwandlungsprozesses bestimmen.

Das genannte Verfahren lässt sich erfindungsgemäss mittels eines Messgerätes durchführen, das mit einem Einlauf und einem Auslauf für das fluide Medium sowie einer in das strömende Medium ragenden Sauerstoffelektrode versehen und dadurch gekennzeichnet ist, dass es eine im Strömungskanal mit der Sauerstoffelektrode angeordnete Pumpe aufweist und der die Sauerstoffelektrode und die Pumpe aufweisende Strömungskanal über steuerbare Ventile wahlweise als Durchflusskanal mit dem Einlauf und Auslauf oder als Ringflusskanal mit einem Überbrückungskanal verbindbar ist. Mit einem solchen Messgerät, das direkt in den Reaktor einbringbar ist, lässt sich sowohl die kontinuierliche Bestimmung des virtuellen Sauerstoffsättigungswertes im Reaktor als auch die dazugehörige Atmungsaktivität des im Reaktor enthaltenen Belebtschlammes messen. Die Pumpe des Messgerätes sorgt dafür, dass bei allen Messungen immer eine ausreichend starke Umströmung der Sauerstoffelektrode durch das fluide Medium stattfindet.

Erfindungsgemäss lässt sich mittels einer zeitlich automatisch arbeitenden Ventilsteuereinrichtung zum Umstellen von der einen auf die andere Messung ein Schliessen oder Öffnen der Ventile des Messgerätes bewirken. Diese Umstellung kann aber auch manuell erfolgen. Alle Messungen erfolgen unter normalen Betriebsbedingungen bei gleichzeitig erhöhter Genauigkeit, was zu einer wesentlichen technischen Verbesserung der bislang bekannten Verfahren und Vorrichtungen auf diesem Gebiete führt.

Der Einlauf des Gerätes lässt sich so ausbilden, dass keine Gasbläschen in das Messgerät gelangen, was eine weitere Erhöhung der Genauigkeit der Sauerstoffkonzentrationsmessung bedeutet. Das Messgerät kann wasserdicht ausgeführt und somit in den Reaktor direkt eingetaucht werden, wodurch Temperatureinflüsse während des Messvorganges ausgeschlossen werden können.

Nachfolgend werden ein Ausführungsbeispiel eines erfindungsgemäss ausgebildeten Messgerätes und die Auswertung der Messergebnisse anhand der beiliegenden Zeichnungen näher erläutert.

Im einzelnen zeigen:
Fig. 1 eine schematische Darstellung des Messgeräts mit seinen wichtigsten Einzelteilen;
Fig. 2 ein zur Auswertung der Messdaten dienendes Diagramm.

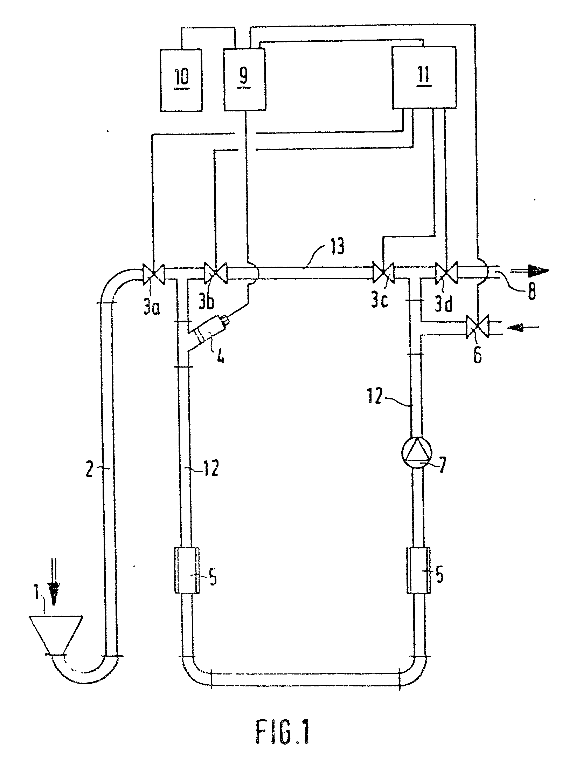

Das in Fig. 1 dargestellte Messgerät weist einen Einlauf 1 auf, der trichterförmig gestaltet ist und in ein U-Rohr 2 übergeht. Durch diese Einlaufgestaltung wird erreicht, dass am Einlauf im eintretenden fluiden Medium enthaltene Gasbläschen entweichen können. Über das U-Rohr 2 und ein erstes Ventil 3a ist der Einlauf 1 mit einem rohrförmigen Strömungskanal 12 verbunden, in welchen eine Sauerstoffelektrode 4 ragt. Der Strömungskanal 12 ist mit Längenausgleichstükken 5 versehen, die eine Änderung seiner Länge erlauben. Im Strömungskanal 12 ist ausserdem eine Pumpe 7 zum Bewegen des fluiden Mediums angeordnet.

Über ein Ventil 3d ist der Strömungskanal 12 mit einem Ausfluss 8 verbunden. An den Strömungskanal 12 ist über ein gesteuertes Ventil 6 ein Zudosierkanal angeschlossen, über welchen Sauerstoff oder $H_2O_2$ zuführbar ist. Mittels eines Überbrückungskanales 13, dessen Enden mit gesteuerten Ventilen 3b und 3c versehen sind, kann der Strömungskanal 12 bei geschlossenen Ventilen 3a und 3d zu einem Ringflusskanal gestaltet werden.

Die Ventile 3a, 3b, 3c und 3d sind an eine Ventilsteuereinrichtung 11 angeschlossen, die ihrerseits von einem Sauerstoffmessteil 9 beeinflusst wird, der mit der Sauerstoffelektrode 4 verbunden ist. An den Sauerstoffmessteil 9 ist ein Schreiber 10 zum laufenden Aufzeichnen der gemessenen Sauerstoffwerte angeschlossen. Der Sauerstoffmessteil 9 beeinflusst ausserdem das Ventil 6 für den Zudosierkanal. Der Sauerstoffmessteil kann mit einer nicht dargestellten Grenzkontaktschaltung versehen sein, die beim Erreichen von Sauerstoffgehalt-Grenzwerten automatisch Schaltungen auslöst. Durch die Längenausgleichstücke 5 ist der Strömungskanal 12 in der Lage, auch als Ringflusskanal bei geschlossenen Ventilen 3a und 3d zusätzliche Dosiermengen aufzunehmen.

Die Wirkungsweise des Messgerätes ist folgende:
Die Ventilsteuereinrichtung 11 schliesst bzw. öffnet paarweise und wechselweise die Ventile 3a, 3d und 3b und 3c. Bei geöffneten Ventilen 3a und 3d und geschlossenen Ventilen 3b und 3c fördert die Pumpe 7 flüssiges Medium aus dem nicht dargestellten Reaktor, in welchen das Messgerät eingetaucht ist, vom Einlauf 1 durch das U-Rohr 2 vorbei an der Sauerstoffelektrode 4 durch den Strömungskanal 12 hindurch zum Auslauf 8. In diesem Falle wirkt der Strömungskanal 12 als Druchflusskanal, und an der Sauerstoffelektrode 4 wird der im durchfliessenden Medium vorhandene Sauerstoffgehalt erfasst und vom angeschlossenen Sauerstoffmessteil 9 mit angeschlossenem Schreiber 10 angezeigt.

Nach einem vorgegebenen Zeitintervall, z.B. einer Stunde, werden durch die Ventilsteuereinrichtung manuell oder automatisch die Ventile 3a und 3d geschlossen und die Ventile 3b und 3c geöffnet. Die zu diesem Zeitpunkt im Strömungskanal 12 des Messgerätes enthaltene Probe an flüssigem Medium wird nun durch die Pumpe 7 über den Überbrückungskanal 13 im Kreislauf durch den Strömungskanal 12 gefördert und dabei laufend an der Sauerstoffelektrode 4 mit kontinuierlicher Strömungsgeschwindigkeit vorbeibewegt. Dabei wird an der Sauerstoffelektrode die durch die im flüssigen Medium enthaltenen Mikroben bedingte Abnahme der Sauerstoffkonzentration erfasst und am Sauerstoffmessteil 9 angezeigt.

Nach dem Verbrauch des in der im Messgerät enthaltenen Probe vorhandenen Sauerstoffes oder nach einer vorgegebenen Messzeit werden, beispielsweise durch einen Sollkontakt der nicht näher dargestellten Grenzkontaktschaltung des Sauerstoffmessteiles 9, die Ventile 3a und 3d durch die Ventilsteuereinrichtung 11 geöffnet und die Ventile 3b und 3c geschlossen. Nun wird mittels der Pumpe 7 erneut flüssiges Medium durch den Einlauf 1 aus dem Reaktor angesaugt, und an der Sauerstoffelektrode 4 wird wieder die Sauerstoffkonzentration im Reaktor erfasst.

Mit den gemessenen virtuellen Sättigungswerten und den dazugehörigen Atmungsaktivitäten der Mikroorganismen kann mit Hilfe einer zu der Versuchsdurchführung passenden Gleichung, z.B. den Gleichungen 3, 4, 6, 8, oder grafisch die Belüftungskonstante bestimmt werden, nach welcher eine Sauerstoffzufuhr in den Reaktor erfolgen soll. Der Sauerstoffeintragswert kann dann mit der Gleichung 5 errechnet werden.

Bei zu geringer Sauerstoffkonzentration im Reaktor muss vor Beginn der Respirationsmessung Reinsauerstoff oder $H_2O_2$ zudosiert werden. Das Messgerät selbst ist wasserdicht und kann vollkommen oder teilweise in Flüssigkeit getaucht werden. Durch eine Änderung der Pumpe 7 und des Rohres 2 kann das Messgerät aber auch so ausgebildet werden, dass es ausserhalb des Reaktors betrieben werden kann.

Das Verfahren zur Auswertung der Messergebnisse basiert auf folgenden Überlegungen:

Geht man von einem sich im Gleichgewicht, auch «steady state» genannten Belebungssystem aus, so gilt

$$k'_La (c'_s - c^*_s) = A_o \qquad \text{(Gl. 7)}$$

Hat man in einem Reaktor zwei Messungen durchgeführt und dabei

$$c^*_1; c^*_{s2}; A_{o1}; A_{o2}$$

ermittelt, so kann man schreiben

$$k'_La (c'_s - c^*_{s1}) - A_{o1} = 0$$

und

$$k'_La (c'_s - c^*_{s2}) - A_{o2} = 0$$

bzw. nach $c'_s$ Elimination

$$k'_La = \frac{A_{o1} - A_{o2}}{c^*_{s2} - c^*_{s1}} \qquad \text{(Gl. 8)}$$

Werden die Schnittpunkte der Geraden mit der Abszisse und Ordinate zur Aufstellung der Gleichung verwandt, so ergibt sich

$$k'_La = \frac{OC-}{\overline{c'_s}} \qquad \text{(Gl. 9)}$$

bzw. $OC' = k'_La \cdot c'_s \qquad \text{(Gl. 5)}$

Weitere mathematische Wege um zu demselben Ergebnis zu kommen sind möglich. Sie werden jedoch hier nicht beschrieben.

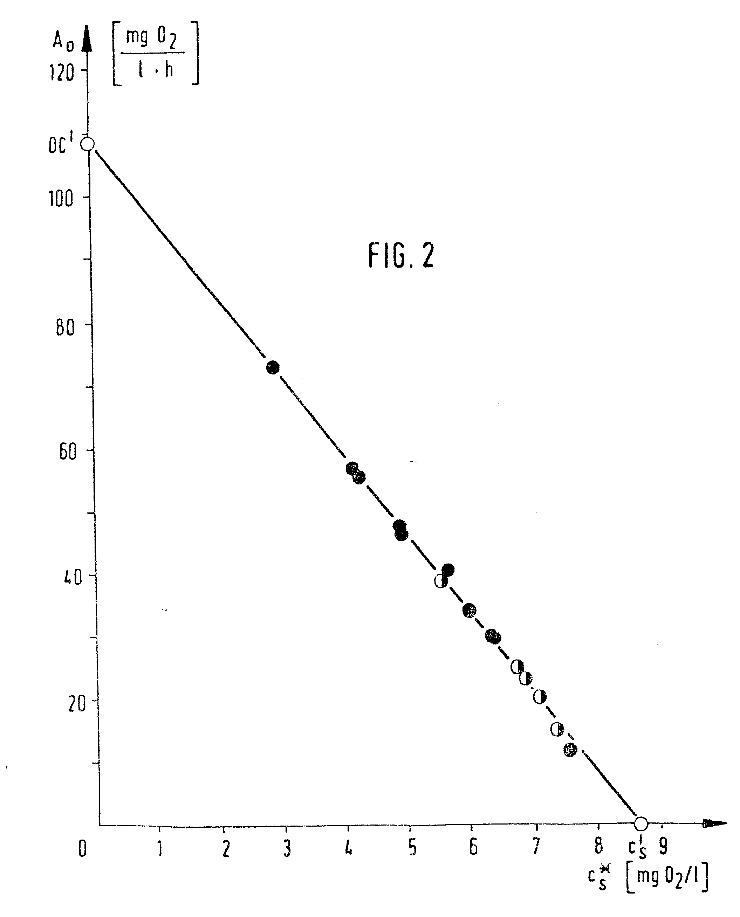

Fig. 2 zeigt ein Diagramm, das eine einfache grafische Auswertung der Messergebnisse und eine direkte Ablesung des Sauerstoffeintragswertes erlaubt, wenn bei unterschiedlichen Belastungszuständen des Reaktors die jeweiligen Sauerstoffkonzentrationswerte (virtuelle Sauerstoffsättigungswerte) und die zugehörigen Respirationswerte in ein karthesisches Koordinatensystem eingetragen werden. Auf der Ordinate des Koordinatensystems wird die Respiration $A_o$ in (mg $O_2$/l h) und auf der Abszisse der virtuelle Sauerstoffsättigungswert $c^*_s$ in (mg $O_2$/l) aufgetragen. Die Messwerte werden durch eine Ausgleichsgerade angenähert. Am Schnittpunkt der Geraden mit der Ordinate ist der gesuchte Sauerstoffeintragswert OC' abzulesen.

## Patentansprüche

1. Verfahren zur Bestimmung des Sauerstoffeintrags in einen mit einem durchmischten fluiden Medium gefüllten Reaktor, dadurch gekennzeichnet, dass im Betrieb des Reaktors abwechselnd der Sauerstoffgehalt des fluiden Mediums und die Sauerstoffgehaltsänderung eines umlaufenden Teiles des fluiden Mediums gemessen werden.

2. Verfahren nach Anspruch 1, dadurch gekennzeichnet, dass beide Messungen bei konstanter und über einem Minimalwert liegender Anströmung einer Sauerstoffelektrode durchgeführt werden.

3. Verfahren nach Anspruch 1, dadurch gekennzeichnet, dass die Messung der Sauerstoffgehaltsänderung nach einer Zudosierung von Reinsauerstoff oder $H_2O_2$ erfolgt.

4. Messgerät zur Bestimmung des Sauerstoffeintrags in einem mit einem durchmischten fluiden Medium gefüllten Reaktor, mit einem Einlauf und einem Auslauf für das fluide Medium sowie einer in das strömende Medium ragenden Sauerstoffelektrode, dadurch gekennzeichnet, dass es eine in dem mit der Sauerstoffelektrode (4) versehenen Strömungskanal (12) angeordnete Pumpe (7) aufweist und der die Sauerstoffelektrode (4) und die Pumpe (7) aufweisende Strömungskanal (12) über steuerbare Ventile (3a, 3b, 3c, 3d) wahlweise als Durchflusskanal mit dem Einlauf (1) und Auslauf (8) oder als Ringflusskanal mit einem Überbrückungskanal (13) verbindbar ist.

5. Messgerät nach Anspruch 4, dadurch gekennzeichnet, dass es eine zeitlich automatisch arbeitende Ventilsteuereinrichtung (11) aufweist.

6. Messgerät nach Anspruch 4, dadurch gekennzeichnet, dass es einen mit einem Schreiber (10) gekoppelten Sauerstoffmessteil (9) aufweist.

7. Messgerät nach Anspruch 4, dadurch gekennzeichnet, dass in den Strömungskanal (12) ein Zudosierkanal über ein durch den Sauerstoffmessteil (9) gesteuertes Ventil (6) mündet.

8. Messgerät nach Anspruch 6, dadurch gekennzeichnet, dass der Sauerstoffmessteil (9) mit einer die Ventilsteuereinrichtung (11) beeinflussenden Grenzkontaktschaltung versehen ist.

9. Messgerät nach Anspruch 4, dadurch gekennzeichnet, dass sein Strömungskanal (12) mit Längenausgleichstücken (5) versehen ist.

## Patent Claims

1. A method of determining the feed of oxygen into a reactor which is filled with a intimately-mixed fluid medium, characterised in that, when the reactor is in operation, alternately the oxygen content of the fluid medium and the change in oxygen content of a circulating part of the fluid medium are measured.

2. A method as claimed in claim 1, characterised in that both measurements are carried out with a constant incident flow, lying above a minimum value, of an oxygen electrode.

3. A method as claimed in claim 1, characterised in that the measurement of the change in the oxygen content is effected after an added dosing of pure oxygen or $H_2O_2$.

4. A measuring instrument for determining the feed of oxygen into a reactor which is filled with an intimately-mixed fluid medium, having an inlet and an outlet for the fluid medium as well as an oxygen electrode which projects into the flowing medium, characterised in that it has a pump (7) which is arranged in the flow duct (12) which is provided with the oxygen electrode (4), and the flow duct (12) which has the oxygen electrode (4) and the pump (7) is connectable by way of controllable valves (3a, 3b, 3c, 3d) selectively as flow-through duct to the inlet (1) and outlet (8) or as ring flow duct to a bridging duct (13).

5. A measuring instrument as claimed in claim 4, characterised in that it has valve control arrangement (11) which operates automatically with respect to time.

6. A measuring instrument as claimed in claim 4, characterised in that it has an oxygen measuring part (9) which is coupled to a recorder (10).

7. A measuring instrument as claimed in claim 4, characterised in that an added-dosing duct opens, by way of a valve (6) which is controlled by the oxygen measuring part (9), into the flow duct (12).

8. A measuring instrument as claimed in claim 6, characterised in that the oxygen measuring part (9) is provided with a limiting contact circuit which affects the valve control arrangement (11).

9. A measuring instrument as claimed in claim 4, characterised in that its flow duct (12) is provided with length compensating pieces (5).

**Revendications**

1. Procédé pour déterminer l'apport d'oxygène dans un réacteur rempli d'un milieu fluide mélangé, caractérisé en ce que, en cours de fonctionnement du réacteur, sont mesurées alternativement la teneur en oxygène du milieu fluide et les variations de la teneur en oxygène d'une partie circulante du milieu fluide.

2. Procédé conforme à la revendication 1, caractérisé en ce que les deux mesures s'effectuent par irrigation constante et supérieure à une valeur minima d'une électrode à oxygène.

3. Procédé conforme à la revendication 1, caractérisé en ce que la mesure de la variation de teneur en oxygène a lieu après un dosage supplémentaire d'oxygène pur ou d'$H_2O_2$.

4. Appareil de mesure pour déterminer l'apport d'oxygène dans un réacteur rempli d'un milieu fluide mélangé, comprenant une entrée et une sortie pour le milieu fluide ainsi qu'une électrode à oxygène plongeant dans le milieu en circulation, caractérisé en ce qu'il comprend une pompe (7) disposée dans le conduit d'écoulement (12) muni de l'électrode à oxygène (4) et en ce que le conduit d'écoulement (12) muni de l'électrode à oxygène (4) et de la pompe (7) est relié sélectivement à des soupapes commandables (3a, 3b, 3c, 3d) pour constituer un conduit de passage entre l'entrée (1) et la sortie (8) ou un conduit en circuit fermé ayant un conduit de pontage (13).

5. Appareil de mesure conforme à la revendication 4, caractérisé en ce qu'il comprend un dispositif de commande de soupapes (11) automatique temporisé.

6. Appareil de mesure conforme à la revendication 4, caractérisé en ce qu'il comprend un organe de mesure d'oxygène (9) couplé avec un enregistreur (10).

7. Appareil de mesure conforme à la revendication 4, caractérisé en ce que dans le conduit d'écoulement (12) débouche une canalisation d'adjonction dosée à travers une soupape (6) commandée par l'organe de mesure d'oxygène (9).

8. Appareil de mesure conforme à la revendication 6, caractérisé en ce que l'organe de mesure d'oxygène (9) est équipé d'un circuit de commutation à contacts limites agissant sur le dispositif de commande de soupapes (11).

9. Appareil de mesure conforme à la revendication 4, caractérisé en ce que le conduit d'écoulement (12) est pourvu de pièces de réglage de la longueur (5).

FIG.1

FIG. 2